(19) Europäisches Patentamt
European Patent Office
Office européen des brevets

(11) **EP 4 108 160 A1**

(12) **EUROPEAN PATENT APPLICATION**

(43) Date of publication:
**28.12.2022 Bulletin 2022/52**

(21) Application number: **21180653.4**

(22) Date of filing: **21.06.2021**

(51) International Patent Classification (IPC):
***A61B 5/00*** (2006.01)  ***A61B 5/0537*** (2021.01)

(52) Cooperative Patent Classification (CPC):
**A61B 5/4881; A61B 5/0537**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Stichting IMEC Nederland**
**5656 AE Eindhoven (NL)**

(72) Inventors:
• **LINDEBOOM, Lucas**
  **Beek en Donk (NL)**
• **GROENENDAAL, Willemijn**
  **Son en Breugel (NL)**
• **HERMELING, Evelien**
  **Soerendonk (NL)**

(74) Representative: **AWA Sweden AB**
**Box 5117**
**200 71 Malmö (SE)**

(54) **A METHOD, A DEVICE, AND A SYSTEM FOR ESTIMATING TOTAL BODY WATER CONTENT**

(57) A method for estimating total body water, TBW, content of a subject, comprises: receiving (202) locally measured bioimpedances for at least two different frequencies of a stimulation current at a body part of the subject; determining (204) a first resistance and a second resistance of the bioimpedance, wherein the first resistance corresponds to a representation of the bioimpedance being purely resistive at a low frequency of the stimulation current and the second resistance corresponds to a representation of the bioimpedance being purely resistive at a high frequency of the stimulation current; and estimating (206) the TBW content of the subject using a ratio between the first resistance and the second resistance.

RECEIVE LOCALLY MEASURED BIOIMPEDANCES FOR AT LEAST TWO DIFFERENT FREQUENCIES OF A STIMULATION CURRENT — 202

DETERMINE A FIRST RESISTANCE AND A SECOND RESISTANCE CORRESPONDING TO THE BIOIMPEDANCE BEING PURELY RESISTIVE AT A LOW FREQUENCY AND A HIGH FREQUENCY , RESPECTIVELY — 204

ESTIMATE TBW CONTENT USING RATIO BETWEEN FIRST RESISTANCE AND SECOND RESISTANCE — 206

*Fig. 4*

**Description**

Technical field

The present inventive concept relates to a method, a device, and a system for estimating total body water (TBW) content of a subject. In particular, the present inventive concept may be used for estimating an absolute amount of TBW content.

Background

Bioimpedance measurements can be used to estimate the absolute amount of TBW content of a subject. Conventionally, the estimation of TBW content is done by using a 5-cylinder model, wherein the body is modelled as five cylindrical conductors in series. Further, a bioimpedance measurement is performed through a whole body (e.g. by arranging electrodes on the ankle and wrist of the subject). Absolute impedance values can be converted into volumes, by using standard available formulas that assume a cylindrical geometry of different body parts. Using multi-frequency bioimpedance measurements, it is also possible to estimate extracellular and intracellular water compartments, separately.

However, performing bioimpedance measurements through the whole body requires a measurement apparatus that provides electrodes being localized in relation to both wrists and ankles, which makes the measurement apparatus inconvenient for use in a setting where the measurement apparatus is worn for a substantial period, such as hours or days. It would be desired to estimate TBW content based on merely a local bioimpedance measurement through a part of the body of the subject. This would enable estimating TBW content using wearable devices that are convenient to wear for a subject and which may anyway be worn by the subject, such as implementing bioimpedance measurements for estimating TBW content in a smartwatch. However, when performing local bioimpedance measurements, the bioimpedance is no longer measured through the whole body and the assumption of a cylindrical geometry of the measured volume does no longer hold and alternative methods to estimate TBW content are needed.

Summary

An objective of the present inventive concept is to provide a method, a device and/or a system for estimating TBW content of a subject based on locally measured bioimpedances. It is particular objective of the present inventive concept to provide a method, a device and/or a system for estimating an absolute amount of TBW content.

These and other objectives of the present inventive concept are at least partly met by the invention as defined in the independent claims. Preferred embodiments are set out in the dependent claims.

According to a first aspect, there is provided a method for estimating total body water, TBW, content of a subject, said method comprising: receiving locally measured bioimpedances for at least two different frequencies of a stimulation current at a body part of the subject; determining a first resistance and a second resistance of the bioimpedance, wherein the first resistance corresponds to a representation of the bioimpedance being purely resistive at a low frequency of the stimulation current and the second resistance corresponds to a representation of the bioimpedance being purely resistive at a high frequency of the stimulation current; and estimating TBW content of the subject using a ratio between the first resistance and the second resistance.

It is an insight of the present inventive concept, that a ratio between the first resistance and the second resistance is dependent on a cell to water fraction in the body of the subject. Further, the first resistance and the second resistance are measured in relation to a common volume, such that when the ratio of the first resistance and the second resistance is considered, the volume through which the bioimpedances are measured can be disregarded. Hence, the cell to water fraction of the body part of the subject may be directly determined from the locally measured bioimpedances using the ratio. This implies that the method enables determination of the TBW content of the subject without requiring measurements to be made through a whole body and without requiring modelling of the body as cylindrical conductors. By the locally measured bioimpedance being representative for the whole body, the locally measured bioimpedances may be used for determining the TBW content of the subject.

The method facilitates performing local bioimpedance measurements using a wearable device. Thus, the measurements may be performed without causing discomfort to the subject wearing the wearable device, such as performing measurements during daily life of the subject. For instance, the local bioimpedance measurements may be performed by a device being worn around a body part, such as in a smartwatch or bracelet, or by a device being worn as a patch on skin of the subject.

Measurements being performed without affecting the daily life of the subject opens up possibilities of estimating and monitoring TBW content in new manners. For instance, continuous monitoring of the TBW content may be provided to improve treatment renal failure and/or end stage renal disease. In alternative applications, TBW content may be monitored as input for dehydration measurements for elderly or for athletes. This may be used for improving elderly care or as input for improving performance by athletes.

The method may simplify acquisition of measured bioimpedances. Since the volume can be disregarded, positioning of electrodes for acquiring the measured bioimpedances is not critical. Hence, a misplacement of an electrode will not affect the estimation of the TBW content.

The method need not be affected by drift of bioimpedance measurements. Drift of bioimpedance measurements may affect both the first resistance and the second resistance, such that the ratio will not be affected. For instance, drift due to ambient temperature may similarly affect the first resistance and the second resistance. This implies that the method is fit for long-term monitoring of TBW content. This facilitates performing continuous monitoring of TBW content using a wearable device.

As used herein, the subject is typically a human being although it should be realized that the method may be applied to an animal.

The first resistance and the second resistance correspond to a representation of the bioimpedance being purely resistive at a low frequency of the stimulation current and a high frequency of the stimulation current, respectively. It should be realized that the bioimpedance may be represented in a complex form having a real part and an imaginary part, wherein the real part corresponds to a resistance and the imaginary part corresponds to a reactance. When the imaginary part of the bioimpedance becomes zero, the reactance is represented merely by the real part and may be considered to be "purely resistive". The bioimpedance may be purely resistive when the frequency of the stimulation current is zero and when the frequency of the stimulation current is infinite. Although it may not be possible to actually measure these bioimpedances, the first resistance and the second resistance may be determined based on the at least two different frequencies used for acquiring the measured bioimpedances. Hence, these determined resistances representing the bioimpedance being purely resistive implies that there is an estimation being performed when determining the respective resistance that represents the bioimpedance being purely resistive. For instance, the resistances may be determined by extrapolation from the measured bioimpedances. The resistances may alternatively be determined by selecting resistances from measured impedances using a low frequency and a high frequency, respectively, if sufficiently disparate frequencies are used, such that the selected resistances may be used as relevant approximations for the first resistance and the second resistance.

The bioimpedances being locally measured implies that the measurement is performed between electrodes that are close to each other, such as within 20 cm or within 10 cm from each other. The local measurement may thus be performed only at a body part, which implies that the electrodes may be integrated in a common device, such as within a patch to be attached to the body part or within a smartwatch. The body part may be any part of the body, such as a limb, a torso, or a neck of the body.

The locally measured bioimpedances may be measured using the same electrodes, such that the measured bioimpedances for the at least two different frequencies are obtained using the same set-up. This implies that the received locally measured bioimpedances represent bioimpedances over the same volume in the body port of the subject.

It should be realized that using of the ratio between the first resistance and the second resistance does not necessarily imply that the ratio is actually calculated. Rather, the ratio could be used such that the relation between the first resistance and the second resistance is utilized for allowing the TBW content to be estimated. Thus, analysis of the locally measured bioimpedances for determining an estimation of the TBW content may be based on the ratio for allowing the estimation to be directly determined from the locally measured bioimpedances. Hence, the TBW content may be estimated from the locally measured bioimpedances without an actual value of the ratio necessarily being determined.

The TBW content may be represented as a fraction of extracellular water compared to a total volume of cells and water or a fraction of intracellular water compared to a total volume of cells and water. Thus, the TBW content may be an indication of a fraction of water within the body. This may be useful as e.g. a measure of (de-)hydration of the subject. However, it should be realized that an absolute amount of TBW content may be estimated. In such case, further input may be necessary for converting the locally measured bioimpedances and a fraction of TBW content estimated thereon to an absolute amount of TBW content.

According to an embodiment, the receiving comprises receiving locally measured bioimpedances for different frequencies of the stimulation current within a range of at least 50 - 500 kHz, such as 10 - 700 kHz, such as 2 kHz - 1 MHz.

This implies that the bioimpedances are measured for a large range of frequencies. The frequencies may be selected to span the entire range, such that at least end points of the range, or frequencies close thereto, are included in the frequencies for which bioimpedances are measured. With measured bioimpedances spanning a large range of frequencies, the first resistance and the second resistance of the bioimpedance being purely resistive may be accurately estimated.

Changes in TBW content may have particularly large effect on bioimpedance for a stimulation current having a frequency around 50 kHz. Therefore, it may be desired that a frequency of 50 kHz is included in the range of frequencies for which the bioimpedance is measured.

The number of different frequencies being used in the measured bioimpedances is at least two. However, many more frequencies may be used, spanning the range of frequencies, e.g. having regular intervals between adjacent frequencies. For instance, ten frequencies or 50 frequencies may be used. It should be realized that, using a larger number of frequencies, an estimation of the relation between bioimpedance and frequency may be improved allowing a high accuracy in estimation of the first resistance and the second resistance. On the other hand, using a larger number of frequencies, a time required for acquisition of the bioimpedance measurements increases and the hardware for generating the frequencies and performing the bioimpedance measurements may be more complicated.

According to an embodiment, the method further comprises performing fitting of the measured bioimpedances to a Havriliak-Negami model to determine the first resistance and the second resistance.

Havriliak-Negami relaxation is a model of dielectric relaxation. Using Havriliak-Negami fitting, the relation between a reactance and a resistance of the measured bioimpedance may be defined in relation to a frequency of the stimulation current.

The reactance may be presented on a y-axis and the resistance may be presented on an x-axis. The Havriliak-Negami fitting may be used for estimating points where the fitting crosses the x-axis, which points represent the bioimpedance being purely resistive (the reactance is zero). Thus, the Havriliak-Negami fitting may be advantageously used for determining the first resistance and the second resistance.

It should be realized that there are variations of the Havriliak-Negami model. For instance, a special case of the Havriliak-Negami model is a Cole-Cole model. In some embodiments, the Havriliak-Negami fitting may be performed as a Cole-Cole fitting.

According to an embodiment, the method further comprises: determining a body cell mass measure representing a body cell mass of the subject; and wherein estimating the TBW content of the subject is configured to estimate an absolute amount of the TBW content using said ratio between the first resistance and the second resistance and based on the body cell mass measure.

Hence, the body cell mass measure may be used for allowing a fraction of extracellular or intracellular water to a total volume of cells and water to be converted to an absolute amount of TBW content. This implies that the absolute amount of TBW content may be determined based merely on locally measured bioimpedances, i.e. it is not necessary to measure the bioimpedance through the whole body.

The body cell mass measure may be determined in various manner. According to an embodiment, said determining of the body cell mass measure comprises at least one of: using a population average based on one or more of gender, height, age or weight; using a measure of potassium content of the subject; using a measure of whole body bioimpedance; or using a calorimetry measure and a relation of resting energy expenditure to body cell mass.

The use of the population average is a simple manner of determining the body cell mass measure, because it does not require any additional measurements to be made. Rather, the body cell mass measure may be estimated from a formula based on one or more of gender, height, weight, or age of the subject.

According to an alternative, the body cell mass measure may be determined from a measurement of potassium-40 content of the subject. The potassium-40 content of the subject may be directly related to the body cell mass of the subject.

According to yet another alternative, a calorimetry measure and a relation of resting energy expenditure to body cell mass may be used. Thus, by receiving a result of a direct or indirect measurement of calorimetry of the subject and using a relation of resting energy expenditure to body cell mass, the calorimetry measure may be converted to the body cell mass measure.

It should be realized that further alternatives for determining the body cell mass measure may be envisaged by the person skilled in the art.

According to an embodiment, the body cell mass measure is used for estimating intracellular water content of the subject.

The body cell mass measure may represent the entire mass of cell matter, including the mass of intracellular water and protein mass. Thus, using the body cell mass measure and a default average fixed density for cells, the volume corresponding to the body cell mass measure may be determined as the body cell mass measure divided by the average fixed density. Further, although body cell mass includes protein mass, the volume of the cells may be considered to correspond to the volume of intracellular water such that the volume corresponding to the body cell mass measure may be considered to represent an estimation of the volume of the intracellular water content of the subject.

According to an embodiment, said ratio between the first resistance and the second resistance is used together with an estimated intracellular water content for estimating extracellular water content of the subject.

Hence, once the intracellular water content is known, the ratio between the first resistance and the second resistance may be used for estimating the extracellular water content.

The total volume of cells and water may be considered to represent the total volume of total body water, since the volume of the cells may be considered to correspond to the volume of intracellular water, as described above. Thus, the fraction of extracellular water or intracellular water compared to a total volume of cells and water may be directly used with the estimated intracellular water content to estimate also the extracellular water content of the subject.

Thus, the method may allow estimating an absolute amount of intracellular water content and extracellular water content, which may further be used for determining a condition of the subject.

According to a second aspect, there is provided a computer program product comprising computer-readable instructions such that when executed on a processing unit the computer program will cause the processing unit to perform the method according to the first aspect.

Effects and features of this second aspect are largely analogous to those described above in connection with the first aspect. Embodiments mentioned in relation to the first aspect are largely compatible with the second aspect.

The computer program product may implement the method in a processing unit, which may be a dedicated processing unit for performing the method or may be a general-purpose processing unit which may be able to perform the method based on the computer program product.

According to a third aspect, there is provided a device for estimating total body water, TBW, content of a subject, said device comprising a processing unit configured to: receive locally measured bioimpedances for at least two different frequencies of a stimulation current at a body part of the subject; determine a first resistance and a second resistance of the bioimpedance, wherein the first resistance corresponds to a representation of the bioimpedance being purely resistive at a low frequency of the stimulation current and the second resistance corresponds to a representation of the bioimpedance being purely resistive at a high frequency of the stimulation current; and estimate the TBW content of the subject using a ratio between the first resistance and the second resistance.

Effects and features of this third aspect are largely analogous to those described above in connection with the first and second aspects. Embodiments mentioned in relation to the first and second aspects are largely compatible with the third aspect.

Thus, the device according to the third aspect may be configured to receive locally measured bioimpedances and is able to provide an estimation of the TBW content based on such measurements. This implies that there is no need for measuring the bioimpedance over the whole body of the subject.

The processing unit may be arranged in a device that is worn by the subject and which may further be configured to acquire locally measured bioimpedances. Alternatively, the processing unit may be arranged in a device that is close to the subject and which may be configured to communicate through wired or wireless communication with a sensor for acquiring the locally measured bioimpedances.

This implies that a result of the estimation of the TBW content may be presented to the subject in the device, being available to the subject. Hence, the estimation of the TBW content may be quickly presented to the subject making estimation of the TBW content available to the subject in real time.

As yet another alternative, the processing unit may be arranged remotely and may be configured to receive the locally measured bioimpedances through communication over a telecommunication or data network, such that the processing unit may for instance be arranged "in the cloud".

According to a fourth aspect, there is provided a system for estimating total body water, TBW, content of a subject, said system comprising: the device according to the third aspect, a bioimpedance measurement unit, which is configured to measure bioimpedances for at least two different frequencies of a stimulation current at a body part of the subject, wherein the bioimpedance measurement unit is configured to transfer the measured bioimpedances to the processing unit of the device.

Effects and features of this fourth aspect are largely analogous to those described above in connection with the first, second and third aspects. Embodiments mentioned in relation to the first, second and third aspects are largely compatible with the fourth aspect.

The system may be configured to acquire measurements as well as to process the measurements for estimating the TBW content. Thus, the system may be self-contained for estimating the TBW content.

The system may be implemented within a single physical housing, such that the processing unit of the device and the bioimpedance measurement unit may be arranged in the same housing. Alternatively, the bioimpedance measurement unit may be separate from the device, wherein the bioimpedance measurement unit is configured to communicate bioimpedance measurements through wired or wireless communication to the device for providing results of the locally measured bioimpedances to the processing unit of the device.

According to an embodiment, the bioimpedance measurement unit comprises a tetrapolar arrangement comprising a first and a second electrode for providing a stimulation current to the subject and a third and a fourth electrode for measuring a voltage therebetween induced by the stimulation current.

This implies that the stimulation current is provided to the subject separately from a measurement of a voltage induced by the stimulation current. Such a set-up allows the bioimpedance measurement to provide a measurement of impedance of the tissue in the subject, which is not affected by an impedance in an interface between skin and electrode.

According to an embodiment, the system comprises a carrier for carrying the bioimpedance measurement unit and the device.

This implies that the bioimpedance measurement unit and the device may be integrated in a single physical unit, which provides a simple interface to the subject, who will only need to handle a single physical unit.

According to an embodiment, the carrier is configured to be worn at or around the body part of the subject.

This implies that the carrier facilitates wearing of the system by the subject. The carrier may be configured such that the system may be worn by the subject, without affecting daily life of the subject, such that estimation of TBW content may be performed while the subject goes on with daily life for continuous monitoring of the TBW content.

The carrier may comprise an adhesive patch carrying the bioimpedance measurement unit and the device. The adhesive patch may be configured for being attached to a body part, such as being attached to a torso or a limb of the subject.

Alternatively, the carrier may be configured to be arranged around a body part. Thus, the carrier may comprise a belt or band that may be arranged around the body part of the subject, such as around a torso or around a limb of the subject.

According to an embodiment, the device is configured to monitor the TBW content of the subject as input for monitoring or treatment of renal failure and/or end stage renal disease.

Patients suffering from renal failure and/or end stage renal disease may require treatment frequently. The patients may be subject to hemodialysis and monitoring of TBW content may allow planning and adjusting of treatment of the patients. Hence, monitoring of TBW content using the system allows treatment to be controlled using bioimpedance measurements that may be acquired in a simple manner and with small or minimal effect on the patient. Further, the monitoring of the TBW content may allow the subject to manage his or her drinking habits, so as to improve the condition of the subject.

According to an embodiment, the device is configured to monitor the TBW content of the subject as input for dehydration measurements for elderly or for athletes.

The estimated TBW content may provide information of (de-)hydration of the subject. This may be used in elderly care for improving the condition of elderly subject. This may also be used for athletes for allowing athletes to adapt to (de-)hydration levels so as to improve performance of the athlete, primarily during practice.

The estimated TBW content may thus be used as input for indicating whether there is a need for the subject to reduce dehydration by drinking.

Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.

Fig. 1 is a schematic view of a system for estimating TBW content according to an embodiment.
Fig. 2 is a schematic view of a graph of reactance versus resistance of the bioimpedance for different frequencies of a stimulation current.
Figs 3a-3b are graphs illustrating reactance versus resistance of the bioimpedance for measurements on a subject and a determined fraction of extracellular water over total body water based on the measurements.
Fig. 4 is a flowchart of a method according to an embodiment.

Detailed description

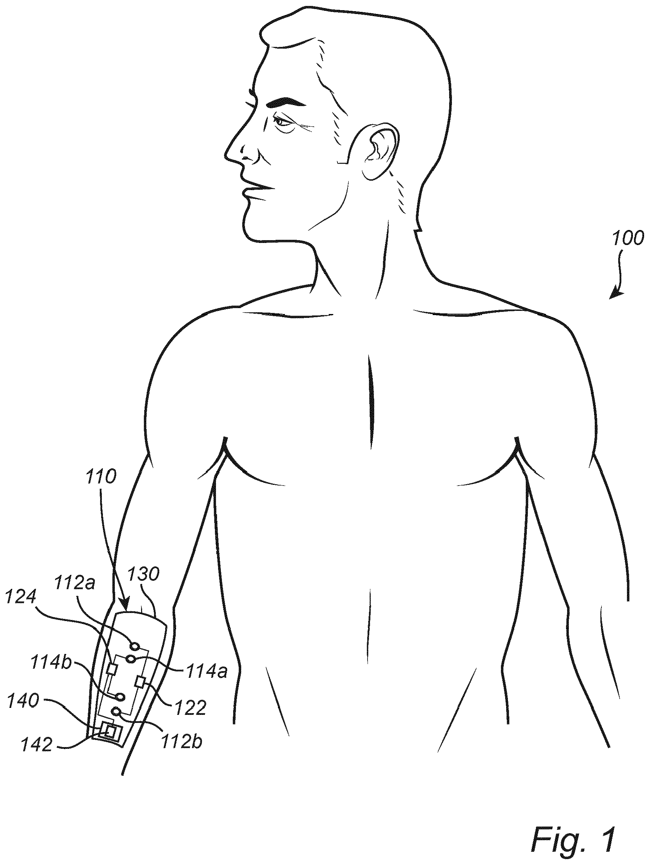

Referring now to Fig. 1, a system 100 for estimating total body water (TBW) content of a subject will initially be briefly described. The system 100 comprises a bioimpedance measurement unit 110, which is configured to measure bioimpedances over a body part of the subject.

In Fig. 1, the bioimpedance measurement unit 110 is illustrated as being arranged for locally measuring bioimpedance at an arm of the subject. The bioimpedance measurement unit 110 comprises stimulation electrodes 112a, 112b, for providing a stimulation current through the body part between the stimulation electrodes 112a, 112b. The bioimpedance measurement unit 110 further comprises measurement electrodes 114a, 114b, for acquiring a signal allowing the voltage across the body part between the measurement electrodes 114a, 114b to be measured.

The signal from the measurement electrodes 114a, 114b may be provided to a measurement circuit 124, which may be configured to determine a bioimpedance based on the measured voltage and the applied stimulation current. The measurement circuit 124 may output a local bioimpedance measurement as a result of the measured voltage and the applied stimulation current. The local bioimpedance measurement may be a digital signal, which may be input to a device 140, comprising a processing unit 142 for estimating TBW content.

The bioimpedance measurement unit 110 may be configured to measure bioimpedances for a plurality, at least two, different frequencies of the stimulation current. The local bioimpedance measurement for each frequency may then be received by the processing unit 142.

The bioimpedance is mainly determined by an amount of water in the body, wherein the water (and dissolved ions) forms a conductive fluid surrounded by nonconductive material of cell membranes. The bioimpedance may be defined in complex form as $Z = R + jX_c$, where Z is the total bioimpedance, R is a resistance forming a real part of the bioimpedance and $X_c$ is a reactance forming an imaginary part of the bioimpedance. Cell membranes act as capacitors contributing to the reactance of the bioimpedance. Since the cell membranes are capacitors, there is no conduction through the capacitor formed by the cell membrane, when the frequency of the stimulation current goes towards zero (a direct current). Hence, fluid within the cells, intracellular water (ICW), is isolated from the stimulation current and the bioimpedance is defined by the fluid outside the cells, extracellular water (ECW). As a frequency of the stimulation current is increased, the impedance of the capacitor formed by the cell membrane decreases and the stimulation current is allowed to flow into the ICW. As the frequency goes towards infinity (or a very high frequency), an effect of the capacitance of the cell membrane is diminished, and the stimulation current is essentially conducted through the TBW, i.e. the ICW and the ECW, defining the bioimpedance.

Based on the above, the bioimpedance for a low frequency (towards zero), may be seen as purely resistive, i.e. the reactance is zero. The bioimpedance may thus have a first resistance, $R_0$, which corresponds to the bioimpedance being purely resistive. Further, the bioimpedance for a high frequency (towards infinity), may also be seen as purely resistive, i.e. the reactance is zero. The bioimpedance may thus have a second resistance, $R\infty$, which also corresponds to the bioimpedance being purely resistive.

The first resistance may be defined as:

$$R_0 = \rho_1 \cdot \frac{L^2}{V},$$

where $\rho_1$ is an apparent resistivity of the ECW, L and V is a length and a volume for the bioimpedance measurement.

Similarly, the second resistance may be defined as:

$$R_\infty = \rho_2 \cdot \frac{L^2}{V},$$

where $\rho_2$ is an apparent resistivity of the TBW, L and V is a length and a volume for the bioimpedance measurement.

It is an insight of the present inventive concept that a ratio between the first resistance and the second resistance is a useful measure. Since the locally measured bioimpedances are measured using a common relation to the body part of the subject, the length and volume for the bioimpedance measurements is the same for the first and second resistances. This implies that a ratio between the first resistance and the second resistance may be written as:

$$\frac{R_0}{R_\infty} = \frac{\rho_1}{\rho_2}. \qquad (Eq. 1)$$

Further, it is an insight of the present inventive concept, that the apparent resistivities of the first resistance and the second resistance may be described in relation to an effect that a concentration of non-conductive material (cell matter) has on the apparent resistivity of the surrounding conductive fluid. Using Bruggeman equations, the following equations may be set up for the first and second resistances, respectively:

$$\frac{\rho_{ECW}}{\rho_1} = (1 - \phi)^{3/2}, \qquad (Eq. 2)$$

where $\rho_{ECW}$ is an actual resistivity of the ECW and $\Phi$ is the volume fraction of cells over the total volume of cells and water and 1- $\Phi$ is thus a corresponding volume fraction of extracellular water over the total volume of cells and water, and

$$\frac{\rho_{ECW} * (\rho_2 - \rho_{ICW})}{\rho_2 * (\rho_{ECW} - \rho_{ICW})} \left( \frac{\rho_2}{\rho_{ECW}} \right)^{1/3} = 1 - \phi, \quad (Eq. 3)$$

where $\rho_{ICW}$ is an actual resistivity of the ICW.

Using equations Eq.1-Eq.3, an equation system of three equations and three unknowns may be set up, such that the three unknowns may be determined. In particular, $\rho_1$, $\rho_2$, and $\Phi$ are unknown, whereas $R_0$ and $R\infty$ are obtained via the locally determined bioimpedance measurements and default values may be used for $\rho_{ECW}$ and $\rho_{ICW}$.

The following default values presented in below Table 1 may be used, depending on gender of the subject:

Table 1: Default values of resistivities of ECW and ICW

|  | $\rho_{ECW}$ | $\rho_{ICW}$ |
|---|---|---|
| Female | 39.0 $\Omega \cdot$cm | 264.9 $\Omega \cdot$cm |
| Male | 40.5 $\Omega \cdot$cm | 273.9 $\Omega \cdot$cm |

The volume of the cells may be considered to correspond to the volume of intracellular water. Thus, the cellular fraction of the total volume of cell matter and fluid in the body could be considered to correspond to a fraction of intracellular water of the total volume of cell matter and fluid in the body. Further, the total volume of cell matter and fluid in the body could be considered to correspond to a volume of TBW in the body. Using the above equations, the extracellular water fraction $1-\Phi$ or intracellular water fraction $\Phi$ may thus be determined, providing an estimate of the TBW content of the subject. In particular, the extracellular water fraction may be used.

The processing unit 142 may be configured to determine the water fraction as an estimate of the TBW content of the subject, by receiving the locally determined bioimpedance measurements for determining the first resistance and the second resistance, as described in further detail below, and by having access to default values of the resistivity of ECW and ICW, respectively.

The extracellular water fraction provides a measure of hydration of the subject, which may in itself be a useful measure, e.g. as an indication of health or condition of the subject.

The extracellular or intracellular water fraction may further be converted to an absolute amount of water in the body of the subject, i.e. an absolute amount of TBW content. This may be achieved using a further input relating to body cell mass of the subject.

According to an embodiment, a body cell mass measure is used for converting the extracellular or intracellular water fraction to an absolute amount of total body water. Body cell mass (BCM) represents a mass of cells, i.e. a sum of protein mass of the cells and a mass of ICW. The BCM is the metabolically active part of the human body and can be determined in several ways.

The BCM measure may be determined using a population average based on one or more of gender, height, age, or weight. Hence, using knowledge of the gender, height, weight and/or age of the subject, the BCM measure may be determined. For instance, the processing unit 142 may have access to stored data relating the BCM measure to one or more of the gender, height, weight and/or age. Thus, values for each of these parameters may be provided to the processing unit 142, e.g. through a user interface for manual input, such that the processing unit 142 may determine the BCM measure through look-up in the stored data or through computation via a formula relating one or more of the parameters to the BCM.

According to an alternative, the body cell mass measure may be determined from a measurement of potassium content of the subject. The potassium content of the subject may be directly related to the BCM of the subject. Potassium-40 is a radioactive isotope of potassium. Thus, a direct measurement of potassium-40 may be achieved by measurement of radioactive radiation from the subject. Hence, the system 100 may comprise a sensor for measuring radioactive radiation from the subject for converting the measurement to potassium-40 content of the subject, which may be further converted to a BCM measure of the subject. However, according to an alternative, a separate measurement of potassium-40 content of the subject may be made such that the system 100 and, in particular the processing device 142, receives the BCM measure based on a potassium-40 measurement from an external device.

According to yet another alternative, a calorimetry measure and a relation of resting energy expenditure to BCM may be used. Thus, by receiving a result of a direct or indirect measurement of calorimetry of the subject, and using a relation of resting energy expenditure to BCM, the calorimetry measure may be converted to the BCM measure.

The system 100 may comprise a calorimetry sensor for direct calorimetry measurement. However, it should be realized that direct calorimetry measurement may require a measurement of amount of heat generated by the body, which sensor needs to accommodate the entire body. Thus, direct calorimetry measurements are complex and require large sensor set-ups. However, according to an alternative, a separate direct calorimetry measurement may be made such that the system 100 and, in particular the processing device 142, receives the BCM measure based on a direct calorimetry measure from an external device.

The system 100 may alternatively comprise an indirect calorimetry sensor set-up for indirect calorimetry measurement. The indirect calorimetry sensor set-up may be configured to measure oxygen consumption, carbon dioxide production and urinary nitrogen loss, through respiratory gas exchange measurement and measurement of urinary nitrogen excretion. However, according to an alternative, a separate direct calorimetry measurement may be made such that the system 100 and, in particular the processing device 142, receives the BCM measure based on an indirect calorimetry measure from an external device.

As mentioned, the BCM measure defines a total mass of cell matter and ICW. Based on the BCM measure, the ICW may be determined using a default average fixed density for cells, by the volume corresponding to the body cell mass measure being determined as the body cell mass measure divided by the average fixed density. Further, as mentioned above, the volume of the cells may be considered to correspond to the volume of ICW such that the volume corresponding to the body cell mass measure may be considered to represent an estimation of the volume of the ICW of the subject.

Further, thanks to the bioimpedance measurements, an extracellular water fraction or intracellular water fraction has been determined, representing ECW / (ECW + ICW) or ICW / (ECW + ICW). Based on any of these fractions, and the estimated volume of ICW through the BCM measure, ECW may be determined. Thereafter, the absolute amount of the TBW content may be determined as a sum of ICW and ECW.

Thus, according to an embodiment, an absolute amount of ICW content is first determined using the BCM measure. Then, using the fraction between ECW and ICW + ECW, wherein the fraction is determined using the ratio of the measured first resistance and second resistance, the absolute amount of ECW content and, hence, the absolute amount of TBW content may also be determined.

It should be realized that the determination of the fraction $\Phi$ or 1- $\Phi$ uses an assumption that the actual resistivities of ECW and ICW are fixed. However, the actual resistivities may be changed if composition (e.g. salt content) changes. Hence, the actual resistivities of either ECW or ICW may change. This would affect the actual resistivities of ECW and ICW as used in the determination of the fraction $\Phi$ or 1- $\Phi$. Therefore, change of composition of ECW or ICW may be a source of error in determination of an absolute amount of TBW. In normal circumstances, it may be assumed that changes in the actual resistivities of ECW or ICW are negligible. However, in special circumstances, such as when a large amount of water is lost true sweat in a short period of time, the source of error may affect the estimation of the absolute amount of TBW.

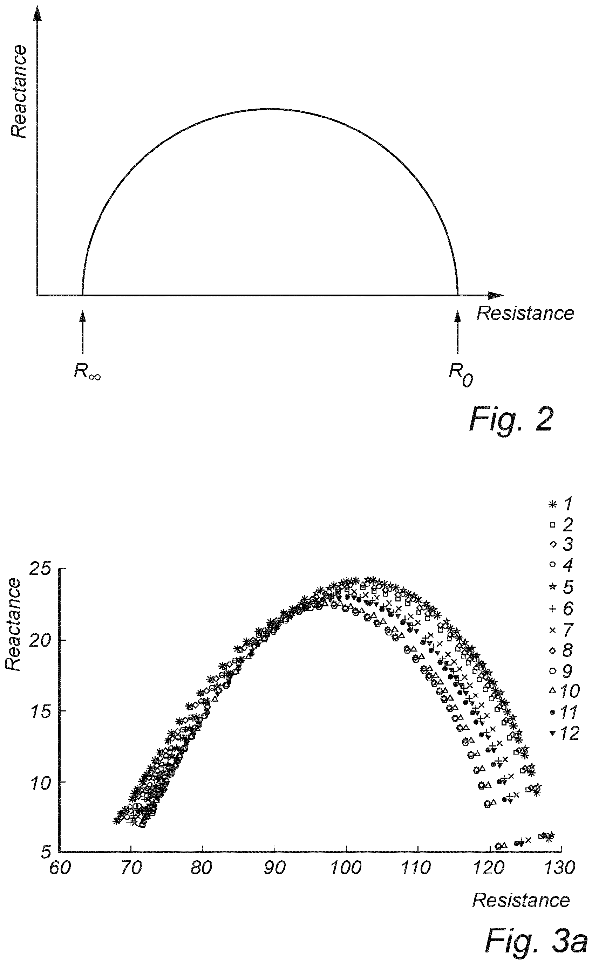

Referring now to Fig. 2, a graph of reactance versus resistance of the bioimpedance will be discussed. As shown in Fig. 2 and further discussed above, the reactance versus resistance is dependent on the frequency of the stimulation current.

For a low frequency, the reactance goes to zero and the resistance is relatively high, since the current may only be conducted through the ECW. As the frequency increases, reactance increases (due to cell membrane capacitance) and the resistance decreases, since the current may now also be conducted through the ICW. Then, as the frequency is further increased, reactance decreases again, as the effect of the capacitance of the cell membrane diminishes, and the resistance continues to decrease, since the current may now be conducted through a larger extent through the ICW. Hence, as shown in Fig. 2, the bioimpedance may be considered as being purely resistive for a low frequency (going towards 0) and for a high frequency (going towards infinity).

As discussed above, the first resistance corresponding to the bioimpedance being purely resistive at a low frequency and the second resistance corresponding to the bioimpedance being purely resistive at a high frequency are to be determined. This may be achieved by performing local bioimpedance measurements for two frequencies, one low frequency and one high frequency, and estimating the first resistance and the second resistance based on these measurements.

However, according to an alternative, the first resistance and the second resistance may be determined by performing a plurality of measurements for different frequencies and fitting the bioimpedance measurements to a curve describing the relation between reactance and resistance so as to allow the first resistance and the second resistance to be determined based on these measurements.

According to an embodiment, bioimpedances may be measured for different frequencies of the stimulation current within a range of at least 50 - 500 kHz, such as 10 - 700 kHz, such as 2 kHz - 1 MHz. This implies that the bioimpedances are measured for a large range of frequencies. The frequencies may be selected to span the entire range, such that at least end points of the range, or frequencies close thereto, are included in the frequencies for which bioimpedances are measured. With measured bioimpedances spanning a large range of frequencies, the first resistance and the second resistance of the bioimpedance being purely resistive may be accurately estimated.

The number of different frequencies being used in the measured bioimpedances is at least two. However, many more frequencies may be used, spanning the range of frequencies, e.g. having regular intervals between adjacent frequencies. For instance, ten frequencies or 50 frequencies may be used.

Since it is in particular the first and second resistances that are of interest, the bioimpedance may be measured for a number of low frequencies and a number of high frequencies, but only few frequencies or no frequencies in-between the high and the low frequencies may be used. Thus, frequencies in a span of 2-10 kHz and frequencies in a span of 700 kHz - 1 MHz may be used for allowing the fitting of the measured bioimpedances to a curve for determining the first resistance and the second resistance.

It should further be realized that other frequencies may alternatively be used, such as frequencies even below 2 kHz or above 1 MHz.

According to an embodiment, the measured bioimpedances may be fitted to a Havriliak-Negami model for determining the first resistance and the second resistance.

The Havriliak-Negami model defines a dielectric relaxation as:

$$\varepsilon^*(\omega) = \varepsilon_\infty + \left[\frac{(\varepsilon_0 - \varepsilon_\infty)}{[1 + (j\omega\tau)^{1-\alpha}]^\beta}\right],$$

where $\varepsilon^*$ in this case corresponds to the bioimpedance, $\omega$ corresponds to the frequency of the stimulation current, $\varepsilon_0$ corresponds to the resistance for a 0 frequency, $\varepsilon_\infty$ corresponds to the resistance for an infinity frequency, $\tau$ is a time constant, $\alpha$ is a parameter defining the curve and $\beta$ is another parameter defining the curve, describing asymmetry and broadness of the curve, respectively. For the special case of $\beta = 1$, the Havriliak-Negami model is called a Cole-Cole model.

The fitting of the bioimpedance measurements to the Havriliak-Negami model may thus be performed for determining values of the parameters defining the model. In particular, the first resistance corresponding to $\varepsilon_0$ and the second resistance corresponding to $\varepsilon_\infty$ may be determined.

The thus determined first resistance and second resistance may then be used with the equations Eq.1-Eq.3 as explained above for determining an estimate of the TBW content.

However, according to an alternative, the fitting of the bioimpedance measurements to the Havriliak-Negami model may be used directly for determining the water fraction in the body of the subject.

In particular, the difference $\varepsilon_0 - \varepsilon_\infty$ as used in the Havriliak-Negami model may be rewritten as:

$$\varepsilon_0 - \varepsilon_\infty = \varepsilon_\infty \frac{\varepsilon_0}{\varepsilon_\infty} - \varepsilon_\infty = \varepsilon_\infty \frac{\frac{\rho_{ECW}}{(1-\phi)^{\frac{3}{2}}}}{\rho_2} - \varepsilon_\infty \quad (Eq.\,4)$$

using Eq.1-Eq.2 above, which could be used for allowing the fraction $1- \varPhi$ to be determined directly from the fitting of the Havriliak-Negami model. Hence, the fitting of the Havriliak-Negami model to the measured bioimpedances may be used for directly determining the water fraction as an estimation of the TBW content, wherein the ratio of the first resistance and the second resistance is used for allowing the fraction to be presented in the difference $\varepsilon_0- \varepsilon_\infty$ as presented in Eq. 4.

Referring now to Figs 3a-3b, results of locally measured bioimpedances over a period of time are illustrated. The locally measured bioimpedances were acquired by arranging stimulation electrodes and measurement electrodes on the arm of a subject. The measurement electrodes were arranged with a distance of 12 cm between the electrodes.

Measurements were performed over 1 hour while the subject was sitting and working at a computer, with measurements being performed every 5 minutes, such that 12 different measurements were acquired. For each measurement, 52 different frequencies were used, ranging from 2 kHz to 1 MHz. Measurement results are illustrated in Fig. 3a, plotting reactance versus resistance for the measured bioimpedances.

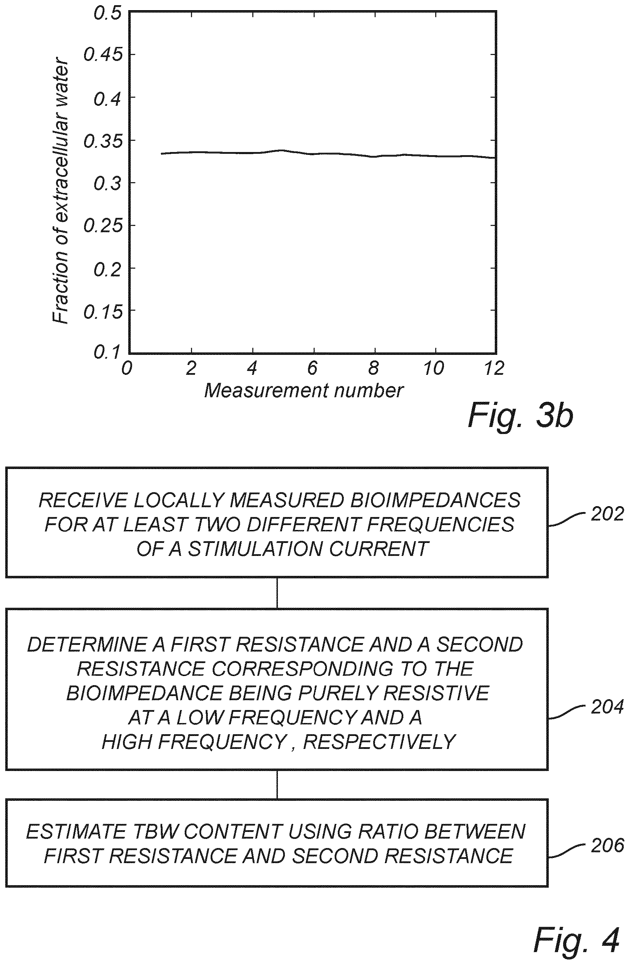

As is evident from Fig. 3a, there is a variation in absolute amounts of measured bioimpedances between the different measurements. In Fig. 3b, the extracellular water fraction as estimated based on the ratio between the first resistance and the second resistance as determined for the different measurements is illustrated. As is evident from Fig. 3b, the extracellular water fraction is stable, even though the absolute impedance is changing +/- 10%. Hence, this illustrates that the use of the ratio between the first resistance and the second resistance can be accurately used for determining the TBW content, even though absolute bioimpedances measured may vary significantly.

Referring now to Fig. 4, the method for estimating TBW content as described above is summarized. The method comprises receiving 202 locally measured bioimpedances for at least two different frequencies of a stimulation current at a body part of the subject. The method further comprises determining 204 the first resistance and the second resistance of the bioimpedance, wherein the first resistance corresponds to a representation of the bioimpedance being purely resistive at a low frequency of the stimulation current and the second resistance corresponds to a representation of the bioimpedance being purely resistive at a high frequency of the stimulation current. The method further comprises estimating 206 the TBW content of the subject using the ratio between the first resistance and the second resistance.

Referring again to Fig. 1, the method may be performed by the processing unit 142 of the device 140. The processing unit 142 may be configured to receive locally measured bioimpedances. The locally measured bioimpedances may be received from the bioimpedance measurement unit 110. The bioimpedance measurement unit 110 may be configured to determine the measured bioimpedances based on signals from the electrodes 114a, 114b, and convert the measured bioimpedances to digital values that may be received by the processing unit.

In this regard, the processing unit 142 does not need any special interface for receiving the bioimpedance measurements and the processing unit 142 may be implemented as a general-purpose processing unit, such as a central processing unit (CPU), which may execute the instructions of one or more computer programs in order to process the received locally measured bioimpedances and to estimate the TBW content. Hence, a computer program product may be provided, which provides computer-readable instructions for causing the processing unit 142 to perform the method. The computer program product may be provided as a signal carrying the computer program product for allowing the computer program product to be loaded into a memory accessible to the processing unit 142. According to an embodiment, the computer program product may be provided as a non-transient computer program product stored on any tangible media.

The processing unit 142 may alternatively be implemented as firmware arranged e.g. in an embedded system, or as a specifically designed processing unit, such as an Application-Specific Integrated Circuit (ASIC) or a Field-Programmable Gate Array (FPGA), which may be configured to implement functionality for processing the received locally measured bioimpedances.

The device 140 comprising the processing unit 142 may for instance be arranged in a wearable device enabling the processing unit 142 to be worn by the subject such that the subject may freely move while measurements are performed and the estimated TBW content is determined. According to an alternative, the processing unit 142 may be arranged in a server "in the cloud" and the bioimpedance measurement unit 110 may be configured to communicate with the processing unit 142 through a telecommunication and/or computer network, such as the Internet.

The bioimpedance measurement unit 110 of the system 100 may be arranged in a wearable device for being worn by the subject, attached to or arranged around a body part of the subject. The wearable device may thus comprise a carrier 130, which is configured to carry the bioimpedance measurement unit 110 such that the carrier 130 allows the bioimpedance measurement unit 110 to be arranged in contact with skin of the subject.

The carrier 130 may comprise an adhesive patch carrying the bioimpedance measurement unit 110. The adhesive patch may be configured for being attached to a body part, such as being attached to a torso or a limb of the subject.

Alternatively, the carrier 130 may be configured to be arranged around a body part. Thus, the carrier 130 may comprise a belt or band that may be arranged around the body part of the subject, such as around a torso or around a limb of the subject.

The device 140 may further be carried by the carrier 130 such that the bioimpedance measurement unit 110 and the device 140 may be integrated in a single physical unit, providing a simple and self-contained system 100.

The bioimpedance measurement unit 110 may comprise, as described above, stimulation electrodes 112a, 112b, for providing a stimulation current through the body part and measurement electrodes 114a, 114b, for acquiring a signal allowing the voltage across the body part between the measurement electrodes 114a, 114b to be measured. Thus, the bioimpedance measurement unit 110 comprises a tetrapolar arrangement for measuring the bioimpedance.

The bioimpedance measurement unit 110 may further comprise a current generator 122 for generating the stimulation current to be provided through the body part of the subject. The current generator 122 may be controlled by a control signal for setting the frequency of the stimulation current to a selected value, such that the bioimpedance may be measured for a plurality of frequencies.

The bioimpedance measurement unit 110 may further comprise a measurement circuit 124 for receiving the signal from the measurement electrodes 114a, 114b. The measurement circuit 124 may determine the voltage as induced by the stimulation current allowing the bioimpedance to be determined based on the induced voltage and the stimulation current. The voltage measured by the measurement circuit 124 may be converted to a digital representation, which may then be used for determining the bioimpedance based on the voltage and the stimulation current.

The system 100 may be useful in various applications. For instance, the device 140 may be configured to monitor the TBW content of the subject as input for monitoring or treatment of renal failure and/or end stage renal disease. The device 140 may thus provide input to a user wearing the system 100 for allowing the user to have knowledge of the TBW content which may greatly affect the condition of the user. For instance, the user may thus use information from the system 100 relating to the TBW content for adjusting drinking habits.

The device 140 may also or alternatively provide information to a caregiver for allowing treatment of patients suffering from renal failure and/or end stage renal disease to be adapted to the TBW content. The patients may be subject to hemodialysis and monitoring of TBW content may allow planning and adjusting of treatment of the patients for improving the treatment of the patients.

In another application, the device 140 is configured to monitor the TBW content of the subject as input for dehydration measurements. Thus, persons that may suffer from dehydration may benefit from information about TBW content, allowing a dehydration condition to be identified such that appropriate measures may be taken.

This may be useful in elderly care, in order to identify when an elderly person is suffering from dehydration or identifying a situation before it becomes critical. The system 100 allows monitoring of the TBW content using a wearable device that may be worn on a body part, such that the system 100 may be compact and may provide a minimal impact on daily life of the subject wearing the system 100.

The monitoring of TBW content as input for detecting dehydration of the subject may also or alternatively be useful for athletes. The system 100 may allow athletes to adapt to dehydration levels so as to improve performance of the athlete, primarily during practice.

The estimated TBW content may thus be used as input for indicating whether there is a need for the subject to reduce dehydration by drinking or by providing fluid replacement.

In the above the inventive concept has mainly been described with reference to a limited number of examples. However, as is readily appreciated by a person skilled in the art, other examples than the ones disclosed above are equally possible within the scope of the inventive concept, as defined by the appended claims.

**Claims**

1. A method for estimating total body water, TBW, content of a subject, said method comprising:

   receiving (202) locally measured bioimpedances for at least two different frequencies of a stimulation current at a body part of the subject;
   determining (204) a first resistance and a second resistance of the bioimpedance, wherein the first resistance corresponds to a representation of the bioimpedance being purely resistive at a low frequency of the stimulation current and the second resistance corresponds to a representation of the bioimpedance being purely resistive at a high frequency of the stimulation current; and
   estimating (206) the TBW content of the subject using a ratio between the first resistance and the second resistance.

2. The method according to claim 1, wherein said receiving (202) comprises receiving locally measured bioimpedances for different frequencies of the stimulation current within a range of at least 50 - 500 kHz, such as 10 - 700 kHz, such as 2 kHz - 1 MHz.

3. The method according to any one of the preceding claims, further comprising performing fitting of the measured bioimpedances to a Havriliak-Negami model to determine the first resistance and the second resistance.

4. The method according to any one of the preceding claims, further comprising:

   determining a body cell mass measure representing a body cell mass of the subject; and
   wherein estimating the TBW content of the subject is configured to estimate an absolute amount of the TBW content using said ratio between the first resistance and the second resistance and based on the body cell mass measure.

5. The method according to claim 4, wherein said determining of the body cell mass measure comprises at least one of: using a population average based on one or more of gender, height, age or weight; using a measure of potassium content of the subject; using a measure of whole body bioimpedance; or using a calorimetry measure and a relation of resting energy expenditure to body cell mass.

6. The method according to any one of claims 4-5, wherein the body cell mass measure is used for estimating intracellular water content of the subject.

7. The method according to claim 6, wherein said ratio between the first resistance and the second resistance is used together with an estimated intracellular water content for estimating extracellular water content of the subject.

8. A computer program product comprising computer-readable instructions such that when executed on a processing unit the computer program will cause the processing unit to perform the method according to any one of the preceding claims.

9. A device (140) for estimating total body water, TBW, content of a subject, said device (140) comprising a processing unit (142) configured to:

   receive locally measured bioimpedances for at least two different frequencies of a stimulation current at a body part of the subject;

determine a first resistance and a second resistance of the bioimpedance, wherein the first resistance corresponds to a representation of the bioimpedance being purely resistive at a low frequency of the stimulation current and the second resistance corresponds to a representation of the bioimpedance being purely resistive at a high frequency of the stimulation current; and

estimate the TBW content of the subject using a ratio between the first resistance and the second resistance.

10. A system (100) for estimating total body water, TBW, content of a subject, said system (100) comprising:

the device (140) according to claim 9,
a bioimpedance measurement unit (110), which is configured to measure bioimpedances for at least two different frequencies of a stimulation current at a body part of the subject, wherein the bioimpedance measurement unit is configured to transfer the measured bioimpedances to the processing unit (142) of the device (140).

11. The system according to claim 10, wherein the bioimpedance measurement unit (110) comprises a tetrapolar arrangement comprising a first and a second electrode (112a, 112b) for providing a stimulation current to the subject and a third and a fourth electrode (114a, 114b) for measuring a voltage therebetween induced by the stimulation current.

12. The system according to claims 10 or 11, further comprising a carrier (130) for carrying the bioimpedance measurement unit (110) and the device (140).

13. The system according to claim 12, wherein the carrier (130) is configured to be worn at or around the body part of the subject.

14. The system according to any one of claims 10-13, wherein the device (140) is configured to monitor the TBW content of the subject as input for monitoring or treatment of renal failure and/or end stage renal disease.

15. The system according to any one of claims 10-13, wherein the device (140) is configured to monitor the TBW content of the subject as input for dehydration measurements for elderly or for athletes.

*Fig. 1*

Fig. 2

Fig. 3a

*Fig. 3b*

RECEIVE LOCALLY MEASURED BIOIMPEDANCES FOR AT LEAST TWO DIFFERENT FREQUENCIES OF A STIMULATION CURRENT — 202

DETERMINE A FIRST RESISTANCE AND A SECOND RESISTANCE CORRESPONDING TO THE BIOIMPEDANCE BEING PURELY RESISTIVE AT A LOW FREQUENCY AND A HIGH FREQUENCY , RESPECTIVELY — 204

ESTIMATE TBW CONTENT USING RATIO BETWEEN FIRST RESISTANCE AND SECOND RESISTANCE — 206

*Fig. 4*

**EP 4 108 160 A1**

Europäisches Patentamt
European Patent Office
Office européen des brevets

# EUROPEAN SEARCH REPORT

Application Number

EP 21 18 0653

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 177 760 A1 (TANITA SEISAKUSHO KK [JP]) 6 February 2002 (2002-02-06) | 1-11,14, 15 | INV. A61B5/00 A61B5/0537 |
| Y | * abstract * <br> * paragraph [0001] * <br> * paragraph [0003] * <br> * paragraph [0008] - paragraph [0011] * <br> * paragraph [0021] - paragraph [0025] * <br> * paragraph [0031] - paragraph [0035] * <br> * paragraph [0053] * <br> * paragraph [0058] - paragraph [0059] * <br> * figures 1-7,12 * | 12,13 | |
| X | EP 1 452 133 A1 (TANITA SEISAKUSHO KK [JP]) 1 September 2004 (2004-09-01) <br> * abstract * <br> * paragraph [0008] - paragraph [0020] * | 1-3,8-10 | |
| Y | US 2019/167208 A1 (LEE YEOL HO [KR] ET AL) 6 June 2019 (2019-06-06) <br> * paragraph [0002] * <br> * paragraph [0007] - paragraph [0019] * <br> * paragraph [0041] - paragraph [0044] * <br> * figures 1-3E * | 12,13 | |
| A | US 2016/058133 A1 (FOURNIER JOSEPH [US]) 3 March 2016 (2016-03-03) <br> * abstract * <br> * paragraph [0058] - paragraph [0059] * <br> * paragraph [0065] * <br> * paragraph [0068] * <br> * paragraph [0075] * <br> * paragraph [0082] - paragraph [0086] * <br> * paragraph [0090] - paragraph [0091] * | 1-3,8-15 | TECHNICAL FIELDS SEARCHED (IPC) <br><br> A61B |
| A | JP H09 51884 A (SEKISUI CHEMICAL CO LTD) 25 February 1997 (1997-02-25) <br> * paragraph [0016] * | 2 | |

-/--

The present search report has been drawn up for all claims

| Place of search | Date of completion of the search | Examiner |
|---|---|---|
| The Hague | 29 November 2021 | Van der Haegen, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

17

Europäisches
Patentamt

European
Patent Office

Office européen
des brevets

# EUROPEAN SEARCH REPORT

Application Number

EP 21 18 0653

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | A.C. Pinheiro Volp et al: "Energy expenditure: components and evaluation methods", Nutrición Hospitalaria, vol. 26, no. 3 1 January 2011 (2011-01-01), pages 430-440, XP055867105, ISSN: 0212-1611, DOI: 10.3305/nh.2011.26.3.5181 Retrieved from the Internet: URL:https://scielo.isciii.es/pdf/nh/v26n3/02_revision_02.pdf [retrieved on 2021-11-29] * the whole document * | 5 | |
| A | LORENZO DE A ET AL: "DETERMINATION OF INTRACELLULAR WATER BY MULTIFREQUENCY BIOELECTRICAL IMPEDANCE", ANNALS OF NUTRITION AND METABOLISM, KARGER, CH, vol. 39, no. 3, 1 January 1995 (1995-01-01), pages 177-184, XP008034499, ISSN: 0373-0101 * the whole document * | 5 | |
| | | | TECHNICAL FIELDS SEARCHED (IPC) |

The present search report has been drawn up for all claims

| Place of search | Date of completion of the search | Examiner |
|---|---|---|
| The Hague | 29 November 2021 | Van der Haegen, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 18 0653

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-2021

| Patent document cited in search report | | | Publication date | Patent family member(s) | | | Publication date |
|---|---|---|---|---|---|---|---|
| EP 1177760 | A1 | | 06-02-2002 | DE | 60129079 | T2 | 28-02-2008 |
| | | | | EP | 1177760 | A1 | 06-02-2002 |
| | | | | JP | 3699640 | B2 | 28-09-2005 |
| | | | | JP | 2002045346 | A | 12-02-2002 |
| | | | | US | 2002022787 | A1 | 21-02-2002 |
| EP 1452133 | A1 | | 01-09-2004 | CN | 1537511 | A | 20-10-2004 |
| | | | | EP | 1452133 | A1 | 01-09-2004 |
| | | | | JP | 2004255120 | A | 16-09-2004 |
| | | | | KR | 20040077558 | A | 04-09-2004 |
| | | | | TW | I270365 | B | 11-01-2007 |
| | | | | US | 2004171963 | A1 | 02-09-2004 |
| | | | | US | 2006229527 | A1 | 12-10-2006 |
| US 2019167208 | A1 | | 06-06-2019 | KR | 20190065086 | A | 11-06-2019 |
| | | | | US | 2019167208 | A1 | 06-06-2019 |
| US 2016058133 | A1 | | 03-03-2016 | US | 2016058133 | A1 | 03-03-2016 |
| | | | | US | 2016226542 | A1 | 04-08-2016 |
| JP H0951884 | A | | 25-02-1997 | JP | 3492038 | B2 | 03-02-2004 |
| | | | | JP | H0951884 | A | 25-02-1997 |

EPO FORM P0459

For more details about this annex : see Official Journal of the European Patent Office, No. 12/82